(19) Europäisches Patentamt / European Patent Office / Office européen des brevets

(11) **EP 3 777 975 A1**

(12) **EUROPEAN PATENT APPLICATION**
published in accordance with Art. 153(4) EPC

(43) Date of publication:
**17.02.2021 Bulletin 2021/07**

(51) Int Cl.:
**A61N 5/10** (2006.01)   **G21K 5/04** (2006.01)

(21) Application number: **19784642.1**

(22) Date of filing: **25.03.2019**

(86) International application number:
**PCT/JP2019/012590**

(87) International publication number:
**WO 2019/198478 (17.10.2019 Gazette 2019/42)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **11.04.2018 JP 2018076463**

(71) Applicant: **NATIONAL INSTITUTES FOR QUANTUM AND RADIOLOGICAL SCIENCE AND TECHNOLOGY**
**Inage-ku**
**Chiba-shi**
**Chiba 263-8555 (JP)**

(72) Inventor: **INANIWA, Taku**
**Chiba-shi, Chiba 263-8555 (JP)**

(74) Representative: **Winter, Brandl, Fürniss, Hübner, Röss, Kaiser, Polte - Partnerschaft mbB**
**Patent- und Rechtsanwaltskanzlei**
**Alois-Steinecker-Straße 22**
**85354 Freising (DE)**

(54) **IRRADIATION PLANNING DEVICE, IRRADIATION PLANNING METHOD, AND CHARGED PARTICLE IRRADIATION SYSTEM**

(57)     Provided are an irradiation planning device capable of accurately predicting the RBE of a combined field in a short time and determining an irradiation parameter, an irradiation planning method, and a charged particle irradiation system. This irradiation planning device 10 is configured so that a storage device 25 of the irradiation planning device 10 stores a domain dose-mean specific energy which is a dose-mean specific energy of domains, a cell nucleus dose-mean specific energy which is a dose-mean specific energy of cell nuclei including a large number of domains, and a domain saturation dose-mean specific energy which is a saturation dose-mean specific energy of the domains, and a computation unit 33 of the irradiation planning device 20 predicts a biological effectiveness at a site of interest from the domain dose-mean specific energy, the cell nucleus dose-mean specific energy, and the domain saturation dose-mean specific energy applied to the site of interest from a pencil beam, and determines the irradiation parameter on the basis of the biological effectiveness.

Fig1

**Description**

TECHNICAL FIELD

The present invention relates to an irradiation planning device that determines an irradiation parameter used by, for example, a charged particle irradiation system which accelerates a charged particle generated by an ion source by an accelerator and irradiates a target with the accelerated charged particle, an irradiation planning method, and a charged particle irradiation system.

BACKGROUND ART

In recent years, a stochastic microdosimetric kinetic model (SMK model, see Non Patent Literatures 1 and 2) has been proposed as a method for estimating the relative biological effectiveness (RBE) of a heavy-ion therapeutic field (combined field). It is known that this SMK model accurately reproduces experimental values including high-dose and high-LET radiation.
This model is a method for estimating, from specific energy delivered to domains and cell nuclei by radiation, the cell killing effect (biological effectiveness) of the radiation. Then, in order to predict the RBE of the combined field according to this model, it is necessary to calculate the profile of the specific energy (specific energy spectrum) delivered to the domains and the cell nuclei by heavy particle beams at each position in the irradiation field. Therefore, in a treatment planning of a scanning irradiation method which requires iteration of iterative approximation calculation, this model requires too much computational time, and thus, it is difficult to apply this model in an actual treatment site.

CITATION LIST

PATENT LITERATURE

Non Patent Literature 1: Tatsuhiko Sato, Yoshiya Furusawa, "Cell survival fraction estimation based on the probability densities of domain and cell nucleus specific energies using improved microdosimetric kinetic models", Radiation research, vol. 178 (2012) pp. 341-356
Non Patent Literature 2: Lorenzo Manganaro, Germano Russo, Roberto Cirio, Federico Dalmasso, Simona Giordanengo, Vincenzo Monaco, Silvia Muraro, Roberto Sacchi, Anna Vignati, Andrea Attili, "A Monte Carlo approach to the microdosimetric kinetic model to account for dose rate time structure effects in ion beam therapy with application in treatment planning simulations", Med. Phys., vol. 44 (2017) pp. 1577-1589

SUMMARY OF THE INVENTION

TECHNICAL PROBLEMS

In view of the above problems, an object of the present invention is to provide an irradiation planning device, an irradiation planning method, and a charged particle irradiation system capable of determining irradiation parameters by accurately predicting RBE of a combined field in a short time.

SOLUTION TO PROBLEMS

The present invention provides: an irradiation planning device including a storage means for storing data, and a computation means for performing computation, the device creating an irradiation parameter used when a charged particle is radiated as a pencil beam, wherein the storage means stores a domain dose-mean specific energy that is a dose-mean specific energy of domains, a cell nucleus dose-mean specific energy that is a dose-mean specific energy of cell nuclei including a large number of domains, and a domain saturation dose-mean specific energy that is a saturation dose-mean specific energy of the domains, and the computation means predicts a biological effectiveness at a site of interest from the domain dose-mean specific energy, the cell nucleus dose-mean specific energy, and the domain saturation dose-mean specific energy applied to the site of interest from the pencil beam, and determines the irradiation parameter on the basis of the biological effectiveness; an irradiation planning method; and a charged particle irradiation system using the same.

ADVANTAGEOUS EFFECTS OF INVENTION

The present invention can provide an irradiation planning device, an irradiation planning method, and a charged particle irradiation system capable of determining irradiation parameters by accurately predicting RBE of a combined field in a short time.

BRIEF DESCRIPTION OF THE DRAWINGS

Fig. 1 is a diagram showing a configuration of a charged particle irradiation system.
Fig. 2 is an explanatory diagram for describing measured cell survival fractions and estimated cell survival fractions.
Fig. 3 is an explanatory diagram of graphs showing dose-mean specific energies $Z^-_{d,D}$, $Z^{-*}_{d,D}$, and $Z^-_{n,D}$ per event.
Fig. 4 is an explanatory diagram for describing d and dose-mean specific energies $Z^-_{d,D}$, $Z^{-*}_{d,D}$, and $Z^-_{n,D}$ as a function of depth for two types of beams.
Fig. 5 is an explanatory diagram for describing d and dose-mean specific energies $Z^-_{d,D}$, $Z^{-*}_{d,D}$, and $Z^-_{n,D}$ at a certain depth for two types of beams.
Fig. 6 is a graph showing a dose-mean specific energy as a function of depth when two beams are superposed.
Fig. 7 is an explanatory diagram showing an image of obtaining a beam axis direction component of pencil beam source data by obtaining a saturation dose-mean specific energy.

DESCRIPTION OF EMBODIMENTS

An embodiment of the present invention will be described below.
The present inventor has conducted an intensive research so that radiation therapy using carbon ions or heavier ions or using a combination of carbon ions or heavier ions and lighter ions is implemented in order to increase the dose of radiation delivered in one treatment and to shorten the treatment period. In addition, the present inventor devises a computing equation that can be computed with higher accuracy than the computation used for a conventional radiation therapy using carbon ions and with a computation speed by which the computing equation can be used in an actual treatment site, and devises an irradiation planning device using the computing equation, and a particle beam irradiation system that radiates particle beams using an irradiation parameter determined by the irradiation planning device.
Hereinafter, the computing equation will be described first, and then, embodiments of the irradiation planning device and the particle beam irradiation system will be described.

«Explanation of computing equation»

<SMK model>

In the SMK model (see Non Patent Literature 1), a cell nucleus can be divided into a number of microscopic sites called domains.
When a population of cells is exposed to ionizing radiation of a macroscopic dose D, specific energy which is the dose absorbed by an individual domain is a random variable that varies from domain to domain throughout the population of cells. Ionizing radiation is assumed to cause two types of lesions in the domain, lethal lesion and sublethal lesion.
A lethal lesion is lethal to the domain including the lesion. A sublethal lesion is non-lethal when generated, and may combine with another sublethal lesion that occurs within the domain to be transformed into a lethal lesion, may spontaneously transform into a lethal lesion, or may be spontaneously repaired. Death of a domain causes inactivation of a cell containing the domain. The number of generated lesions is proportional to the saturation specific energy $z'_d$ in the domain given by [Equation 1].

[Equation 1]

$$z'_d = z_0 \sqrt{1 - \exp\left[-(z_d/z_0)^2\right]}$$

where $z_0$ is a saturation parameter that represents the reduction in the number of complex DNA damages per dose in a high LET region (Hada and Georgakilas 2008 *1).

*1: Hada M and Georgakilas AG 2008 Formation of clustered DNA damage after high-LET irradiation: a review J.

Radial. Res. 49 203-210

Considering the time variation of the lesions, the survival fraction $s_d$ of a domain receiving $z_d$ is determined as the probability that there are no lethal lesions in the domain, and its natural logarithm is calculated by [Equation 2].

[Equation 2]

$$\ln s_d\left(z_d\right) = -Az_d' - Bz_d'^2$$

where A and B are parameters independent of the energy delivered by radiation. Assuming that the cell nucleus contains p domains, the natural logarithm of the survival fraction $S_n$ of the cell that receives a cell-nucleus specific energy $z_n$ can be expressed by [Equation 3].

[Equation 3]

$$\ln S_n\left(z_n\right) = \sum_{i=1}^{p} \ln s_d\left(z_d\right) = -\alpha_0 \int_0^\infty z_d' f_d\left(z_d, z_n\right) dz_d - \beta_0 \int_0^\infty z_d'^2 f_d\left(z_d, z_n\right) dz_d$$

where $\alpha_0$ and $\beta_0$ are pA and pB, respectively, which represent $\alpha$ and $\beta$ parameters in the LQ model in the limit of LET = 0. $f_d(z_d, z_n)$ is the probability density of the domain specific energy $z_d$ within the cell nucleus that receives the cell-nucleus specific energy $z_n$.

In consideration of the stochastic variation of $z_n$ within a population of cells, the natural logarithm of the survival fraction of cells exposed to D is calculated by [Equation 4].

[Equation 4]

$$\ln S(D) = \ln\left[\int_0^\infty S_n\left(z_n\right) f_n\left(z_n, D\right) dz_n\right]$$

where $f_n(z_n, D)$ is the probability density of $z_n$ within the population of cells exposed to the macroscopic dose D, and has the following relations expressed in [Equation 5] and [Equation 6].

[Equation 5]

$$\int_0^\infty z_n f_n\left(z_n, D\right) dz_n = D$$

[Equation 6]

$$\int_0^\infty z_n^2 f_n\left(z_n, D\right) dz_n = \bar{z}_{n,D} D + D^2$$

$Z_{n,D}^-$ is the dose-mean specific energy per event absorbed by a cell nucleus, and is calculated by [Equation 7] together with $z_n$ and a single-event probability density $f_{n,1}(z_n)$.

[Equation 7]

$$\bar{z}_{n,D} = \frac{\int_0^\infty z_n^2 f_{n,1}(z_n)\,dz_n}{\int_0^\infty z_n f_{n,1}(z_n)\,dz_n}$$

Non Patent Literature 1 describes that a computation model for calculating $f_d(z_d, z_n)$ and $f_n(z_n, D)$ under a given irradiation condition is developed, and [Equation 3] and [Equation 4] are numerically solved. Calculations of $f_d(z_d, z_n)$ and $f_n(z_n, D)$ for multiple-event irradiation require macroscopic beam transport simulation by Monte Carlo method for each irradiation condition, and further requires convolution integration of the single-event probability densities $f_{d,1}(z_n)$ and $f_{n,1}(z_n)$ of $z_d$ and $z_n$, and therefore, they need a lot of computational time.

In Non Patent Literature 1, for the calculation of $f_d(z_d, z_n)$, the convolution integration of $f_{d,1}(z_d)$ is further omitted, assuming that the saturation effect triggered by multiple radiation events to a domain is negligibly small. Then, $\ln S_n$ can be simplified as expressed in [Equation 8].

[Equation 8]

$$\ln S_n(z_n) = -\alpha_0 z_n \frac{\bar{z}_{d,F}^*}{\bar{z}_{d,F}} - \beta_0 \left( \bar{z}_{d,D} z_n + z_n^2 \right) \frac{\bar{z}_{d,D}^*}{\bar{z}_{d,D}}$$

The frequency-mean specific energy per event is expressed by [Equation 9].

[Equation 9]

$$\bar{z}_{d,F} = \int_0^\infty z_d f_{d,1}(z_d)\,dz_d$$

The saturation frequency-mean specific energy per event is expressed by [Equation 10].

[Equation 10]

$$\bar{z}_{d,F}^* = \int_0^\infty z_d' f_{d,1}(z_d)\,dz_d$$

The dose-mean specific energy per event is expressed by [Equation 11].

[Equation 11]

$$\bar{z}_{d,D} = \frac{\int_0^\infty z_d^2 f_{d,1}(z_d)dz_d}{\int_0^\infty z_d f_{d,1}(z_d)dz_d} = \frac{\int_0^\infty z_d^2 f_{d,1}(z_d)dz_d}{\bar{z}_{d,F}}$$

The saturation dose-mean specific energy per event is expressed by [Equation 12].

[Equation 12]

$$\bar{z}_{d,D}^* = \frac{\int_0^\infty z_d'^2 f_{d,1}(z_d)dz_d}{\bar{z}_{d,F}}$$

Next, [Equation 8] and [Equation 4] are the basic equations of the SMK model that are solved to estimate the survival fraction of cells. In Non Patent Literature 1, cell survival fractions are estimated using the SMK model, and it is found that they are generally in close agreement with those estimated by directly solving [Equation 3] and [Equation 4] for the dose ranges used in most charged-particle therapy (for example, the absorbed dose of D being equal to or less than 10 Gy for a therapeutic carbon-ion beam).

The conventional calculation based on the SMK model requires the convolution integration of $f_{n,1}(z_n)$ for deriving $f_n(z_n,D)$ as well as the macroscopic beam transport simulation by a Monte Carlo method for each treatment radiation. Therefore, in order to apply this model to a daily treatment planning in which a survival fraction at each location needs to be predicted for each patient, extensive computation is still demanded. In addition, in order to adapt the SMK model to a treatment planning of a scanned charged-particle therapy, $f_{n,1}(z_n)$ at respective locations for beamlets of all spots across a target volume needs to be stored in a memory space, which requires a huge memory area.

The stored $f_{n,1}(z_n)$ is then used to update $f_n(z_n, D)$ at each location of the field in every iteration of iterative approximation. Thus, the adaptation of the SMK model to the treatment therapy of scanned charged-particle therapy is especially difficult both in time and memory space, at least with computers commonly used for commercialized treatment planning systems.

In view of the problem in which the conventional SMK model cannot be applied to a particle radiation therapy device which emits a scanning beam, the present inventors have devised a novel model to be described below as a <modified SMK model> to address the problem.

<Modified SMK model>

In charged particle therapy, the domain specific energy $z_d$ is generally delivered by a great number of low-energy transfer events, and events that induce saturation of complex DNA damages are rare. In other words, events that induce $z_d > z_o$ are rare.

Therefore, the approximation expressed by [Equation 13] is established for the frequency-mean specific energy.

[Equation 13]

$$\bar{z}_{d,F}^* \Big/ \bar{z}_{d,F} \approx 1$$

Then, [Equation 8] can be rearranged as [Equation 14] using [Equation 15] and [Equation 16].

[Equation 14]

$$\ln S_n(z_n) = -\left(\alpha_0 + \overline{z}_{d,D}^* \beta_0\right) z_n - \beta_0 \frac{\overline{z}_{d,D}^*}{\overline{z}_{d,D}} z_n^2$$

$$\equiv -\alpha_{SMK} z_n - \beta_{SMK} z_n^2$$

[Equation 15]

$$\alpha_{SMK} \equiv \left(\alpha_0 + \overline{z}_{d,D}^* \beta_0\right)$$

[Equation 16]

$$\beta_{SMK} \equiv \beta_0 \left(\overline{z}_{d,D}^* / \overline{z}_{d,D}\right)$$

If [Equation 14] is written in the form of survival fractions, it is rewritten as [Equation 17].

[Equation 17]

$$S_n(z_n) = \exp\left(-\alpha_{SMK} z_n - \beta_{SMK} z_n^2\right)$$

Assuming that the number of energy transfer events to a cell nucleus is large, the second-order Taylor expansion of $S_n$ around $z_n = D$ can be expressed by [Equation 18] using [Equation 19], [Equation 20], and [Equation 21].

[Equation 18]

$$S_n(z_n) \approx \exp\left(-\alpha_{SMK} D - \beta_{SMK} D^2\right)$$
$$- \left(\alpha_{SMK} + 2\beta_{SMK} D\right) \exp\left(-\alpha_{SMK} D - \beta_{SMK} D^2\right)(z_n - D)$$
$$+ \left[-\beta_{SMK} + \frac{1}{2}\left(\alpha_{SMK} + 2\beta_{SMK} D\right)^2\right] \exp\left(-\alpha_{SMK} D - \beta_{SMK} D^2\right)(z_n - D)^2$$
$$\equiv \exp\left(-\alpha_{SMK} D - \beta_{SMK} D^2\right)\left[G_0 + G_1 z_n + G_2 z_n^2\right]$$

[Equation 19]

$$G_0 = 1 + \alpha_{SMK} D + \left(\beta_{SMK} + \frac{1}{2}\alpha_{SMK}^2\right) D^2 + 2\alpha_{SMK}\beta_{SMK} D^3 + 2\beta_{SMK}^2 D^4$$

[Equation 20]

$$G_1 = -\alpha_{\text{SMK}} - \alpha_{\text{SMK}}^2 D - 4\alpha_{\text{SMK}}\beta_{\text{SMK}} D^2 - 4\beta_{\text{SMK}}^2 D^3$$

[Equation 21]

$$G_2 = -\beta_{\text{SMK}} + \frac{1}{2}\alpha_{\text{SMK}}^2 + 2\alpha_{\text{SMK}}\beta_{\text{SMK}} D + 2\beta_{\text{SMK}}^2 D^2$$

By substituting [Equation 18] into [Equation 4], the survival fraction of cells exposed to D is calculated by [Equation 22] using the relationship between [Equation 5] and [Equation 6].

[Equation 22]

$$\ln S(D) = \ln\left[\int_0^\infty \exp\left(-\alpha_{\text{SMK}} D - \beta_{\text{SMK}} D^2\right)\left[G_0 + G_1 z_n + G_2 z_n^2\right]f_n\left(z_n, D\right)dz_n\right]$$

$$= \ln\left[\exp\left(-\alpha_{\text{SMK}} D - \beta_{\text{SMK}} D^2\right)\left\{G_0 + G_1\int_0^\infty z_n f_n\left(z_n, D\right)dz_n + G_2\int_0^\infty z_n^2 f_n\left(z_n, D\right)dz_n\right\}\right]$$

$$= \ln\left[\exp\left(-\alpha_{\text{SMK}} D - \beta_{\text{SMK}} D^2\right)\left\{G_0 + \left(G_1 + G_2\bar{z}_{n,D}\right)D + G_2 D^2\right\}\right]$$

Then, lnS can be rewritten to [Equation 23] using [Equation 19] to [Equation 21], or can be rewritten to [Equation 24] in the form of cell survival fraction.

[Equation 23]

$$\ln S(D) = \ln\left[1 + D\left\{-\beta_{\text{SMK}} + \frac{1}{2}\left(\alpha_{\text{SMK}} + 2\beta_{\text{SMK}} D\right)^2\right\}\bar{z}_{n,D}\right] - \alpha_{\text{SMK}} D - \beta_{\text{SMK}} D^2$$

[Equation 24]

$$S(D) = \exp\left(-\alpha_{\text{SMK}} D - \beta_{\text{SMK}} D^2\right)\left[1 + D\left\{-\beta_{\text{SMK}} + \frac{1}{2}\left(\alpha_{\text{SMK}} + 2\beta_{\text{SMK}} D\right)^2\right\}\bar{z}_{n,D}\right]$$

<Determination of parameters of modified SMK model>

In the modified SMK model, the parameters required to estimate the cell survival fraction are $\alpha_0$ in [Equation 15], $\beta_0$ in [Equation 16], the saturation parameter zo in [Equation 1], the radius $r_d$ of the domain, and the radius $R_n$ of the cell nucleus. $r_d$ is used to derive $Z^-_{d,D}$ and $Z^{-*}_{d,D}$, and $R_n$ is used to derive $Z^-_{n,D}$. These parameters depend only on the type or state of a cell to be used, and are independent of the type of radiation. Among the parameters, the radius $R_n$ can be measured directly with an optical microscope.

It is to be noted that, for "$Z^-_{d,D}$, $Z^-_{n,D}$, $Z^{-*}_{d,D}$", a bar indicating the average value should normally be attached above "z" as shown in Equations 15 and 16. However, since the bar cannot be expressed in the text of the specification, it is displayed as "Z-". Hereinafter, "Z-" in the text of the present specification means that a bar is written above "z" in all equations.

It is reported that, as a result of measurement of $R_n$ of 16 human cell lines, $R_n$ ranges from 6.7 pm to 9.5 pm with the average around 8.1 pm (Suzuki et al 2000 *2).

*2: Suzuki M, Kase Y, Yamaguchi H, Kanai T and Ando K 2000 Relative biological effectiveness for cell-killing effect on various human cell lines irradiated with heavy-ion medical accelerator in chiba (HIMAC) carbon-ion beams Int. J Radiat. Oneal. Biol. Phys. 48 241-250

Since the estimated cell survival fraction is insensitive to $R_n$ (see Non Patent Literature 1), it is assumed that Ro is 8.1 pm for all human cell lines in order to reduce the number of parameters to be determined for SMK calculation.
Numerical values of the other parameters are determined by comparing estimated and measured cell survival fractions under various irradiation conditions by least-square regression.
In the modified SMK model, a cell survival fraction is calculated from the dose-mean specific energies $Z^-_{d,D}$ and $Z^{-*}_{d,D}$ per event applied to the domain and the dose-mean specific energy $Z^-_{n,D}$ per event applied to the cell nucleus, using [Equation 24]. Specific energy is obtained according to a known calculation procedure (Inaniwa et al 2010 *3).
*3: Inaniwa T, Furukawa T, Kase Y, Matsufuji N, Toshito T, Matsumoto Y, Furusawa Y and Noda K 2010 Treatment planning for a scanned carbon ion beam with a modified microdosimetric kinetic model. Phys. Med. Biol. 55 6721-37
The sensitive volumes of the domain and cell nucleus are assumed as cylinders of water having radii $r_d$ and $R_n$ and lengths $2r_d$ and $2R_n$, respectively. Incident ions traverse around the sensitive volumes with uniform probability, and their paths are always parallel to the cylindrical axis. The radial dose distribution around the trajectory of the ions is described by the Kiefer-Chatterjee amorphous ion track structure model (Chatterjee and Schaefer 1976 *4, Kiefer and Straaten 1986 *5, Kase et al 2006 *6).

*4: Chatterjee A and Schaefer H J 1976 Microdosimetric structure of heavy ion tracks in tissue Radial. Environ. Biophys. 13 215-227
*5: Kiefer J and Straaten H 1986 A model of ion track structure based on classical collision dynamics. Phys. Med. Biol. 311201-1209
*6: Kase Y, Kanai T, Matsumoto Y, Furusawa Y, Okamoto H, Asaba T, Sakama M and Shinoda H 2006 Microdosimetric measurements and estimation of human cell survival for heavy-ion beams Radial. Res. 166 629-638

For the comparison with the measured cell survival fractions, a monoenergetic spectrum of $^3$He-, $^{12}$C- and $^{20}$Ne-ion beams with reported LETs is assumed, unlike the actual experiments where $^3$He-, $^{12}$C- and $^{20}$Ne-ion beams with incident energies of 12 and 135 MeV/u were degraded by polymethyl-methacrylate plates of different thicknesses (Furusawa et al 2000 *7).

*7: Furusawa Y, Fukutsu K, Aoki M, Itsukaichi H, Eguchi-Kasai K, Ohara H, Yatagai F, Kanai T and Ando K 2000 Inactivation of aerobic and hypoxic cells from three different cell lines by accelerated He-, C- and Ne-ion beams Radial. Res. 154 485-96

This assumption is reasonable because the effects of inelastic scattering as well as the energy struggling by the plates on $Z^-_{d,D}$, $Z^{-*}_{d,D}$, and $Z^-_{n,D}$ are insignificant for the low-energy beams.
In the least-square regression, parameter values by which the total square deviation of $\log_{10}S$ calculated by [Equation 25] is minimized by changing the SMK parameters other than $R_n$ in a stepwise manner are determined as optimum values for the parameters.

[Equation 25]

$$\chi^2 = \sum_{i=1}^{n} \left[ \log_{10}\left(S_{i,\exp}\right) - \log_{10}\left(S_{i,\mathrm{cal}}\right) \right]^2$$

where $S_{i,\exp}$ and $S_{i,\mathrm{cal}}$ are the measured and survival fractions estimated under the i-th irradiation condition, and the number n of survival fraction data used in the regression for each cell line is approximately 300.
The parameters of the known MK model are determined separately to reproduce the same in vitro experimental data for HSG and V79 cells. The MK model parameters are determined according to the procedures described in Inaniwa et al 2010 *3, except that the measured cell survival fraction data for $^3$He-, $^{12}$C- and $^{20}$Ne-ion beams are directly used in the present embodiment rather than 10%-survival doses derived from the data.

<Calculation of biological dose based on modified SMK model>

Assuming that the survival curve of a reference radiation, i.e., a 200-kV x-ray, follows the LQ model even at extremely high doses, the biological dose at location x can be expressed by [Equation 26].

[Equation 26]

$$D_{\mathrm{bio}}(\mathbf{x}) = D(\mathbf{x}) \cdot RBE(\mathbf{x}) = \frac{-\alpha_X + \sqrt{\alpha_X^2 - 4\beta_X \ln S(D(\mathbf{x}))}}{2\beta_X}$$

where $\alpha_x$ and $\beta_x$ are the linear and quadratic coefficients of the LQ model of the reference radiation. With the modified SMK model, the natural logarithm lnS(D) of the survival fraction in a field (combined field) of therapeutic charged particle beams at the location x is calculated by [Equation 27].

[Equation 27]

$$\ln S(D) = \ln\left[1 + D\left\{-\beta_{\mathrm{SMK}} + \frac{1}{2}(\alpha_{\mathrm{SMK}} + 2\beta_{\mathrm{SMK}}D)^2\right\}\bar{z}_{n,D}\right] - \alpha_{\mathrm{SMK}}D - \beta_{\mathrm{SMK}}D^2$$

$$= \ln\left[1 + D\left\{-\beta_0\frac{\bar{z}^*_{\mathrm{d,D,mix}}}{\bar{z}_{\mathrm{d,D,mix}}} + \frac{1}{2}\left(\alpha_0 + \beta_0\bar{z}^*_{\mathrm{d,D,mix}} + 2\beta_0\frac{\bar{z}^*_{\mathrm{d,D,mix}}}{\bar{z}_{\mathrm{d,D,mix}}}D\right)^2\right\}\bar{z}_{n,D,mix}\right]$$

$$-\left(\alpha_0 + \beta_0\bar{z}^*_{\mathrm{d,D,mix}}\right)D - \beta_0\frac{\bar{z}^*_{\mathrm{d,D,mix}}}{\bar{z}_{\mathrm{d,D,mix}}}D^2$$

where $\bar{z}_{\mathrm{d,D,mix}}$ and $\bar{z}^*_{\mathrm{d,D,mix}}$ are the dose-mean specific energy and saturation dose-mean specific energy per event absorbed by the domain, and $\bar{z}_{\mathrm{n,D,mix}}$ is the dose-mean specific energy per event absorbed by the cell nucleus in the combined field.

For the scanned charged-particle therapy, the absorbed dose D at x is given by [Equation 28].

[Equation 28]

$$D = \sum_j d_j \cdot w_j$$

where $d_j$ is the dose applied by a scanning pencil beam of the jth spot (jth beamlet) to x, and $w_j$ is the number of incident ions of the jth beamlet.

The specific energies at x in the combined field are described as follows.

[Equation 29]

$$\bar{z}_{d,D,mix} = \frac{\int_0^\infty z_d^2 f_{d,1}(z_d)dz_d}{\int_0^\infty z_d f_{d,1}(z_d)dz_d} = \frac{\sum_j d_j \cdot \bar{z}_{d,D,j} \cdot w_j}{\sum_j d_j \cdot w_j}$$

[Equation 30]

$$\bar{z}_{d,D,mix}^* = \frac{\int_0^\infty z_d'^2 f_{d,1}(z_d)dz_d}{\int_0^\infty z_d f_{d,1}(z_d)dz_d} = \frac{\sum_j d_j \cdot \bar{z}_{d,D,j}^* \cdot w_j}{\sum_j d_j \cdot w_j}$$

[Equation 31]

$$\bar{z}_{n,D,mix} = \frac{\int_0^\infty z_n^2 f_{n,1}(z_n)dz_n}{\int_0^\infty z_n f_{n,1}(z_n)dz_n} = \frac{\sum_j d_j \cdot \bar{z}_{n,D,j} \cdot w_j}{\sum_j d_j \cdot w_j}$$

where $Z_{d,D,j}^-$ and $Z_{d,D,j}^{-*}$ are the dose-mean specific energy and the saturation dose-mean specific energy per event of the domain applied by the jth beamlet to x, and $Z_{d,D,j}^-$ is the dose-mean specific energy per event of the cell nucleus applied by the jth beamlet to x.

In scanned charged particle therapy treatment planning, the number of particles w for all beamlets needs to be determined by iteration of an iterative approximation calculation in order to achieve a desired biological dose distribution for a patient. The analytical solution (see [Equation 36] described later) of the gradient of the biological dose with respect to the number of particles $w_j$ is used in the iteration calculation algorithm of interactive approximation calculation, for instance, in the quasi-Newton method. This will be described in detail in the appendix below.

<Pencil beam data>

In order to calculate the biological dose distribution described in [Equation 26] using D, $Z_{d,D,mix}^-$, $Z_{d,D,mix}^{-*}$, and $Z_{n,D,mix}^-$ obtained from [Equation 28] to [Equation 31] in the treatment planning of scanned charged-particle therapy, it is necessary to calculate the quantities $d_j$, $Z_{d,D,j}^-$, $Z_{d,D,j}^{-*}$, and $Z_{n,D,j}^-$ for each beamlet.

In the treatment planning software, distributions of d, $Z_{d,D}^-$, $Z_{d,D}^{-*}$, and $Z_{n,D}^-$ for pencil beams in water are determined in advance, and registered in the treatment planning software as pencil beam data. The data is applied to patient dose calculations with density scaling using the stopping-power ratio of body tissues to water to calculate the quantities of $d_j$, $Z_{d,D,j}^-$, $Z_{d,D,j}^{-*}$, and $Z_{n,D,j}^-$ of the jth beamlet in each treatment plan.

The distributions of d, $Z_{d,D}^-$, $Z_{d,D}^{-*}$, and $Z_{n,D}^-$ of the pencil beam in the water can be obtained from all ion tracks, which deliver energy to x, of the pencil beam in [Equation 32], [Equation 33], [Equation 34], and [Equation 35], respectively.

[Equation 32]

$$d = \sum_k e_k$$

[Equation 33]

$$\bar{z}_{d,D} = \frac{\sum_k e_k \cdot \left(\bar{z}_{d,D}\right)_k}{\sum_k e_k}$$

[Equation 34]

$$\bar{z}^*_{d,D} = \frac{\sum_k e_k \cdot \left(\bar{z}^*_{d,D}\right)_k}{\sum_k e_k}$$

[Equation 35]

$$\bar{z}_{n,D} = \frac{\sum_k e_k \cdot \left(\bar{z}_{n,D}\right)_k}{\sum_k e_k}$$

In these Equations, $e_k$ is the energy applied to x by the kth track of the pencil beam. $(Z^-_{d,D})_k$ and $(Z^{-*}_{d,D})_k$ are the dose-mean specific energy and the saturation dose-mean specific energy per event of the domain at x by the kth ion track, respectively, and obtained by the abovementioned method assuming an amorphous ion track structure and cylindrical domain.

$(Z^-_{n,D})_k$ is the dose-mean specific energy per event of the cell nucleus at x by the kth track. The values of $e_k$, $(Z^-_{d,D})_k$, $(Z^{-*}_{d,D})_k$, and $(Z^-_{n,D})_k$ are obtained using a Monte Carlo beam transport simulation of an ion beam simulating the irradiation system in each facility.

<Beam transport simulation>

As therapeutic beams in charged particle therapy, $^4$He-, $^{12}$C-, and $^{20}$Ne-ions have been or will be used. Therefore, in this numerical calculation study, those three species are selected as ion species for the therapeutic beams. The Monte Carlo software PTSim is used to derive $e_k$, $(Z^-_{d,D})_k$, $(Z^{-*}_{d,D})_k$, and $(Z^-_{n,D})_k$ of the ion pencil beams (Aso et al 2007 *8).

*8: Aso T, Kimura A, Kameoka S, Murakami K, Sasaki T and Yamashita T 2007 Geant4 based simulation framework for particle therapy system. Nuclear Science Symposium IEEE, Conference Record 4 2564-2567

This is a particle therapy simulation code built with the Geant4 toolkit (version 9.2 and patch 01) (Agostinelli et al 2003 *9).

*9: Agostinelli Set al. 2003 Geant4 - a simulation toolkit. Nucl. Jnstrum. Methods. Phys. Res. A 506 250-303

The package of physical interactions, the model of the scanning irradiation system, and the geometry of the water phantom used in the Monte Carlo simulation are the same as those described in Inaniwa et al. (2017)*10.

*10: Inaniwa T, Kanematsu N, Noda Kand Kamada T 2017 Treatment planning of intensity modulated composite particle therapy with dose and linear energy transfer optimization Phys. Med. Biol. 62 5180-97

In the simulations, three ion species with initial energies Eo corresponding to the water equivalent ranges from 10 mm to 300 mm in 2 mm steps, i.e., 146 energies, are generated just upstream of scanning magnets with a 2D symmetric Gaussian profile with 2 mm standard deviation. The generated ions pass through the scanning radiation system and enter a water phantom of $200 \times 200 \times 400$ mm$^3$. The phantom volume is divided into $1.0 \times 1.0 \times 0.5$ mm$^3$ units (referred to as "voxels") to record the spatial distributions of various quantities of the simulated ions, namely ion species defined by the mass number Ap and the atomic number Zp, voxel location, kinetic energy T of the ion, and energy e applied to the voxel.

The dose distributions d of the pencil beams are simply derived by [Equation 32] from the recorded distribution of e. To efficiently derive the distributions of $Z^-_{d,D}$, $Z^{-+}_{d,D}$, and $Z^-_{n,D}$ for the beams of [Equation 33], [Equation 34], and [Equation 35], respectively, from the recorded quantities of $Z_p$, T, and e, $Z^-_{d,D}$, $Z^{-*}_{d,D}$, and $Z^-_{n,D}$ for monoenergetic ions with $Z_p$ = 1 - 10 are tabulated as a function of their kinetic energy T.

<Analytical beam modeling>

The fitting procedures described in Inaniwa and Kanematsu (2015) *11 are applied to the simulated distributions of dose d and specific energies $Z^-_{d,D}$, $Z^{-*}_{d,D}$, and $Z^-_{n,D}$ to construct a triple-Gaussian trichrome beam model.

*11: Inaniwa T and Kanematsu N 2015 A trichrome beam model for biological dose calculation in scanned carbon-ion radiotherapy treatment planning Phys. Med. Biol. 60 437-451

In the beam model, the transverse dose profile of the beam is represented as the superposition of three Gaussian distributions, and different specific energies are assigned to the three Gaussian components to represent the radial variation of the specific energies over the beam cross section. For $^{12}$C- and $^{20}$Ne-ion beams, $Z^-_{d,D}$, $Z^{-*}_{d,D}$, and $Z^-_{n,D}$ of the primary ions, the heavy fragments with the atomic number $Z_p \cong 3$ and light fragments with $Z_p \leq 2$ are assigned to the first, second, and third Gaussian components, respectively. For $^4$He ion beam, $Z^-_{d,D}$, $Z^{-*}_{d,D}$, and $Z^-_{n,D}$ of the primary ions are assigned to the first component, while those of other fragments are assigned to the second and third Gaussian components (Inaniwa et al 2017 *10).

With this analytical beam model, effects of large-angle scattered particles on the dose and specific energy distribution are accounted for in the treatment planning using a pencil beam algorithm.

When the beam model was constructed in this way and then applied to a clinical case, it was confirmed that the irradiation parameters could be determined through computation in a significantly shorter time than the conventional method, and the accuracy was also higher. Therefore, after the following appendix, an embodiment of the irradiation planning device and a particle beam irradiation system using the irradiation planning device will be described.

<Appendix>

The analytical solution of $(\nabla D_{bio})_J$ of [Equation 36] at the position x in the combined radiation field of the therapeutic charged particle beam is written as [Equation 37].

[Equation 36]

$$\left( \nabla D_{\mathrm{bio}} \right)_j = \partial D_{\mathrm{bio}} / \partial w_j$$

[Equation 37]

$$\left(\nabla D_{\text{bio}}\right)_j = \frac{\partial D_{\text{bio}}}{\partial w_j} = \frac{\partial \ln S(D)}{\partial w_j} \frac{\partial D_{\text{bio}}}{\partial \ln S(D)}$$

$$= -\frac{\dfrac{\partial \ln S(D)}{\partial w_j}}{2\beta_X \sqrt{\left(\dfrac{\alpha_X}{2\beta_X}\right)^2 - \dfrac{\ln S(D)}{\beta_X}}}$$

The derivative of lnS(D) with respect to $w_j$ is given by [Equation 38], [Equation 39], [Equation 40], [Equation 41], and [Equation 42].

[Equation 38]

$$\frac{\partial \ln S(D)}{\partial w_j} = \left\{ 1 + \left[ -\beta_0 \frac{\overline{Z}_{d,D}^*}{\overline{Z}_{d,D}} + \frac{1}{2}\left( \alpha_0 + \beta_0 \frac{\overline{Z}_{d,D}^*}{D} + 2\beta_0 \frac{\overline{Z}_{d,D}^*}{\overline{Z}_{d,D}} D \right)^2 \right] \overline{Z}_{n,D} \right\}^{-1}$$

$$\times \left[ \begin{array}{l} \left( 4\beta_0^2 \dfrac{D\overline{Z}_{d,D}^{*2}\overline{Z}_{n,D}^2}{\overline{Z}_{d,D}^2} - \alpha_0\beta_0 \dfrac{\overline{Z}_{d,D}^*\overline{Z}_{n,D}}{D^2} + 2\alpha_0\beta_0 \dfrac{\overline{Z}_{d,D}^*\overline{Z}_{n,D}}{\overline{Z}_{d,D}} - \beta_0^2 \dfrac{\overline{Z}_{d,D}^{*2}\overline{Z}_{n,D}}{D^3} \right) d_j \\[12pt] + \left( \beta_0 \dfrac{\overline{Z}_{d,D}^*\overline{Z}_{n,D}}{\overline{Z}_{d,D}^2} - 4\beta_0^2 \dfrac{D^2\overline{Z}_{d,D}^{*2}\overline{Z}_{n,D}^2}{\overline{Z}_{d,D}^3} - 2\alpha_0\beta_0 \dfrac{D\overline{Z}_{d,D}^*\overline{Z}_{n,D}}{\overline{Z}_{d,D}^2} - 2\beta_0^2 \dfrac{\overline{Z}_{d,D}^{*2}\overline{Z}_{n,D}}{\overline{Z}_{d,D}^2} \right) \overline{z}_{d,D,j} d_j \\[12pt] + \left( -\beta_0 \dfrac{\overline{Z}_{n,D}}{\overline{Z}_{d,D}} + \beta_0^2 \dfrac{\overline{Z}_{d,D}^*\overline{Z}_{n,D}}{D^2} + 4\beta_0^2 \dfrac{D^2\overline{Z}_{d,D}^*\overline{Z}_{n,D}^2}{\overline{Z}_{d,D}^2} + \alpha_0\beta_0 \dfrac{\overline{Z}_{n,D}}{D} + 2\alpha_0\beta_0 \dfrac{D\overline{Z}_{n,D}}{\overline{Z}_{d,D}} + 4\beta_0^2 \dfrac{\overline{Z}_{d,D}^*\overline{Z}_{n,D}}{\overline{Z}_{d,D}} \right) \overline{z}_{d,D,j}^* d_j \\[12pt] + \left( -\beta_0 \dfrac{\overline{Z}_{d,D}^*}{\overline{Z}_{d,D}} + \dfrac{\alpha_0^2}{2} + \dfrac{\beta_0^2}{2} \dfrac{\overline{Z}_{d,D}^{*2}}{D^2} + 4\beta_0^2 \dfrac{D^2\overline{Z}_{d,D}^{*2}\overline{Z}_{n,D}}{\overline{Z}_{d,D}^2} + \alpha_0\beta_0 \dfrac{\overline{Z}_{d,D}^*}{D} + 2\alpha_0\beta_0 \dfrac{D\overline{Z}_{d,D}^*}{\overline{Z}_{d,D}} + 2\beta_0^2 \dfrac{\overline{Z}_{d,D}^{*2}}{\overline{Z}_{d,D}} \right) \overline{z}_{n,D,j} d_j \end{array} \right]$$

$$- \left( \alpha_0 + 4\beta_0 \frac{D\overline{Z}_{d,D}^*}{\overline{Z}_{d,D}} \right) d_j + 2\beta_0 \frac{D^2\overline{Z}_{d,D}^*}{\overline{Z}_{d,D}^2} \overline{z}_{d,D,j} d_j - \beta_0 \left( 1 + 2\frac{D^2}{\overline{Z}_{d,D}} \right) \overline{z}_{d,D,j}^* d_j$$

[Equation 39]

$$D = \sum_{j'} d_{j'} \cdot w_{j'}$$

[Equation 40]

$$\overline{Z}_{\mathrm{d,D}} = \sum_{j'} d_{j'} \cdot \overline{z}_{\mathrm{d,D},j'} \cdot w_{j'}$$

[Equation 41]

$$\overline{Z}_{\mathrm{d,D}}^{*} = \sum_{j'} d_{j'} \cdot \overline{z}_{\mathrm{d,D},j'}^{*} \cdot w_{j'}$$

[Equation 42]

$$\overline{Z}_{\mathrm{n,D}} = \sum_{j'} d_{j'} \cdot \overline{z}_{\mathrm{n,D},j'} \cdot w_{j'}$$

Next, as one embodiment of the present invention, an example using the abovementioned computing equation will be described with reference to the drawings.

Example

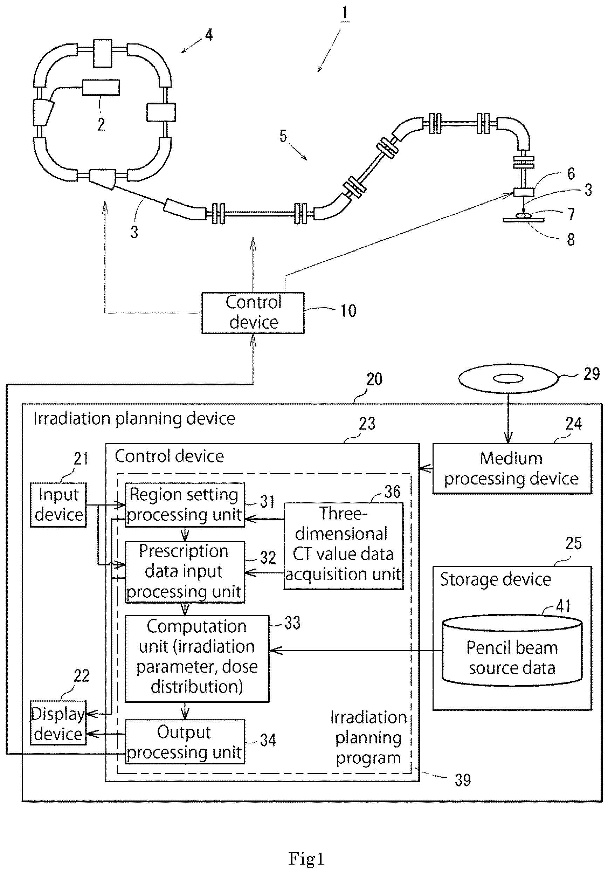

Fig. 1 is a diagram showing a system configuration of a particle beam irradiation system 1.

The particle beam irradiation system 1 includes: an accelerator 4 for accelerating and emitting a charged particle beam 3 emitted from an ion source 2; a beam transport system 5 for transporting the charged particle beam 3 emitted from the accelerator 4; an irradiation device (scanning irradiation device) 6 for irradiating a target part 8 (for example, a tumor part) that is an irradiation target of a patient 7 with the charged particle beam 3 that has passed through the beam transport system 5; a control device 10 that controls the particle beam irradiation system 1; and an irradiation planning device 20 as a computer that determines an irradiation parameter of the particle beam irradiation system 1. The present example shows a case in which carbon and helium are used as nuclides (ion species) of the charged particle beam 3 emitted from the ion source 2, but the present invention is not limited thereto. The present invention is applicable to the particle beam irradiation system 1 that emits various particle beams having neon, oxygen, protons, and the like as nuclides (ion species). Further, although the particle beam irradiation system 1 uses a spot scanning method, a system using another scanning irradiation method such as a raster scanning method may be used.

The accelerator 4 adjusts the intensity of the charged particle beam 3.

The irradiation device 6 includes: a scanning magnet (not shown) for deflecting the charged particle beam 3 in XY directions that define a plane perpendicular to the beam traveling direction (Z direction); a dose monitor (not shown) for monitoring the position of the charged particle beam 3; and a range shifter (not shown) for adjusting the stop position of the charged particle beam 3 in the Z direction, and scans the target part 8 with the charged particle beam 3 along a scan trajectory.

The control device 10 controls the intensity of the charged particle beam 3 from the accelerator 4, the position correction of the charged particle beam 3 in the beam transport system 5, the scanning by the scanning magnet (not shown) of the irradiation device 6, the beam stop position by the range shifter (not shown), etc.

The irradiation planning device 20 includes: an input device 21 including a keyboard and a mouse; a display device 22 including a liquid crystal display or a CRT display; a control device 23 including a CPU, a ROM, and a RAM; a medium processing device 24 including a disk drive or the like for reading and writing data from and to a storage medium 29 such as a CD-ROM and a DVD-ROM; and a storage device 25 (storage means) including a hard disk or the like.

The control device 23 reads an irradiation planning program 39 stored in the storage device 25, and functions as a region setting processing unit 31, a prescription data input processing unit 32, a computation unit 33 (computation means), an output processing unit 34, and a three-dimensional CT value data acquisition unit 36.

The storage unit 25 stores pencil beam source data 41 preset for each radionuclide (for each ion species). The pencil beam source data 41 has, as information of the pencil beam in the beam axis direction, a depth dose distribution d, dose-mean specific energies of domain and cell nucleus ($Z^{-}_{\mathrm{d,D}}$, $Z^{-}_{\mathrm{n,D}}$), and a saturation dose-mean specific energy of domain ($Z^{-*}_{d,D}$) in water, which are obtained in advance.

In the irradiation planning device 20 configured as described above, each functional unit operates as follows according to the irradiation planning program 39.

First, the three-dimensional CT value data acquisition unit 36 acquires three-dimensional CT value data of an irradiation target (patient) from another CT device.

The region setting processing unit 31 displays the image of the three-dimensional CT value data on the display device 22, and receives a region designation (designation of the target part 8) input by a plan creator using the input device 21.

The prescription data input processing unit 32 displays a prescription input screen on the display device 22, and receives prescription data input by the plan creator using the input device 21. This prescription data consists of the irradiation position of a particle beam at each coordinate of the three-dimensional CT value data, the survival fraction (or clinical dose equivalent thereto) desired at that irradiation position, the irradiation direction of the beam, and the type (nuclide) of the particle beam. Further, various settings such as a setting to minimize the influence on the periphery of the irradiation position are input as prescription data.

The computation unit 33 receives the prescription data and the pencil beam source data 41, and creates an irradiation parameter and a dose distribution on the basis of the received data. That is, in order to perform irradiation by which the irradiation target (for example, a tumor such as a cancer cell) at the irradiation position of the prescription data has the survival fraction in the prescription data, the irradiation parameter of the particle beam emitted from the particle beam irradiation system 1 is calculated by calculating back the type and amount (number of particles) of the particle beam to be emitted from the particle beam irradiation system 1 using the pencil beam source data 41. This calculation will be described later.

The output processing unit 34 outputs and displays the calculated irradiation parameter and dose distribution on the display device 22. Further, the output processing unit 34 transmits the irradiation parameter and the dose distribution to the control device 10 that controls the particle beam irradiation system 1.

Next, a method of creating the pencil beam source data 41 will be described in detail.

The pencil beam source data 41 is determined by [Equation 32], [Equation 33], [Equation 34], and [Equation 35] described above from the spatial distribution of e, Zp, and T acquired by Monte Carlo simulation. It is to be noted that, for simplification of [Equation 33], [Equation 34], and [Equation 35], table data is created for each nuclide and stored in the storage device 25.

Table data for each nuclide shows that how much energy is applied when a certain nuclide is radiated with a certain kinetic energy (or speed) for the domain dose-mean specific energy $Z^-_{d,D}$, the domain saturation dose-mean specific energy $Z^{-*}_{d,D}$, and the cell nucleus dose-mean specific energy $Z^-_{n,D}$.

This table is created in advance as follows using [Equation 24].

That is, the parameters required to estimate the cell survival fraction S(D) using [Equation 24] are $\alpha_0$ in [Equation 15], $\beta_0$ in [Equation 16], the saturation parameter zo in [Equation 1], the radius $r_d$ of the domain, and the radius $R_n$ of the cell nucleus. Here, since the radius $R_n$ of the cell nucleus can be directly measured by an optical microscope, it is given by the measurement. Therefore, the values that need to be determined are $\alpha_0$, $\beta_0$, saturation parameter $z_0$, and domain radius $r_d$. Note that $\alpha_0$, $\beta_0$, $z_0$, and $r_d$ are parameters that are determined depending on the cell species.

Therefore, for multiple types of nuclides that can be used in the particle beam irradiation system 1, the cell survival fraction when each nuclide is radiated multiple times with different energies is measured for each nuclide, and the optimum $\alpha_0$, $\beta_0$, saturation parameter $z_0$, and radius of $r_d$ of domain are derived by an appropriate optimization method such as the least square method so that the deviations between the measured values and the calculated values calculated using [Equation 24] are minimized for all nuclides (multiple types of nuclides) and all energies (multiple different energies).

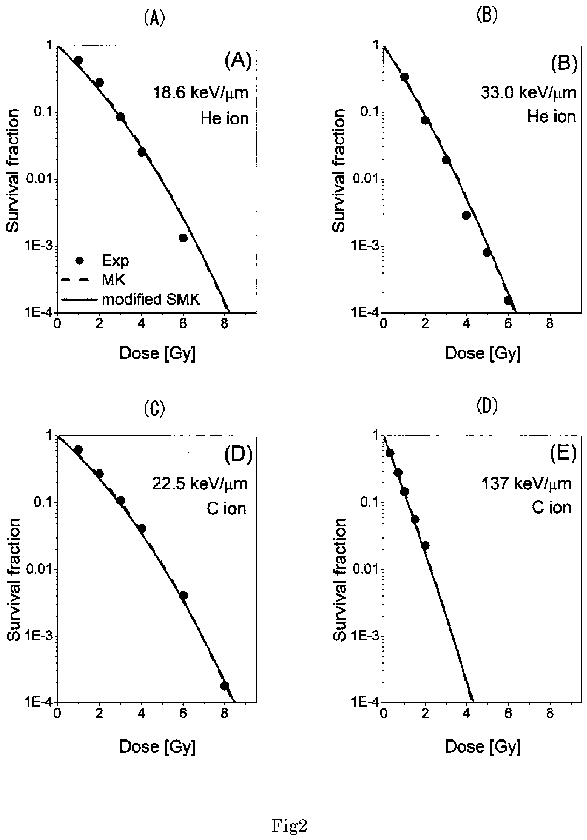

Fig. 2 is an explanatory diagram of graphs in which the measured values and the calculated values are approximated by the modified SMK model in the manner described above, and Figs. 2(A) to 2(D) are graphs with vertical axes indicating a survival fraction and horizontal axes indicating an irradiation dose.

Figs. 2(A) and 2(B) show measured values (black circles in the figures) and calculated values (solid line in the figures) for a certain cell species when helium ion is used as a nuclide, and Figs. 2(C) and 2(D) show measured values (black circles in the figures) and calculated values (solid line in the figures) for a certain cell species (same as the cell species in Figs. 2(A) and 2(B)) when carbon ion is used as a nuclide. Further, Fig. 2(A) shows the case where radiation is delivered with LET = 18.6 keV/$\mu$m, Fig. 2(B) shows the case with LET = 33.00 keV/$\mu$m, Fig. 2(C) shows the case with LET = 22.5 keV/$\mu$m, and Fig. 2(D) shows the case with LET = 137 keV/$\mu$m.

In this way, a set of ($\alpha 0$, $\beta 0$, z0, rd) is determined by which the calculated value (survival fraction) obtained by [Equation 24] for a certain cell species accurately reproduces the measured value (survival fraction) when all types of radiations having different LETs are radiated by varying a dose using all types of nuclides that are used in the particle beam irradiation system 1.

Fig. 2 shows examples of the measured and estimated cell-survival fractions of HSG and V79 cells, respectively,

exposed to $^3$He- and $^{12}$C-ion beams over wide dose and LET ranges. Note that the dotted lines in each of Figs. 2(A) to 2(D) show the case where the calculated values are obtained using the conventional MK model. Although the difference appears to be small in the figure, a significant difference appears when, for example, carbon ions are radiated at 333 keV/$\mu$m or neon ions are radiated at 654 keV/$\mu$m.

In this way, when $\alpha_0$, $\beta_0$, $z_0$, and $r_d$ are calculated for each cell species so that the deviation between the measured value and the calculated value is minimized even if the nuclide, its LET, and dose are varied, the variables $\alpha_0$, $\beta_0$, $z_0$, and $r_d$, which are variables in [Equation 24], are determined for each cell species as fixed parameters. Therefore, if the dose D at the irradiation position is determined for all nuclides, the survival fraction S(D) at that irradiation position can be predicted by [Equation 24].

The measured value is obtained by multiple (preferably, four or more, more preferably, five or more, and most preferably, six) measurements with the dose being varied within a range where the survival fraction is greater than 0 and less than 1. The dose for obtaining the measured value for each nuclide is set such that the survival fraction is 0.1 or more (preferably, 0.3 or more) when a dose with the highest survival fraction is applied, and the survival fraction is 0.05 or less (preferably, 0.03 or less) when a dose with the lowest survival fraction is applied. Further, the dose is varied within the range so as not to concentrate on one side.

Although Fig. 2 shows the values at two types of LETs, it is preferable to determine a set of ($\alpha0$, $\beta0$, $z0$, $rd$) by which the measured values and the calculated values are accurately reproduced over a wider LET.

Table 1 below shows examples of fixed parameters in the modified SMK model determined to reproduce the measured cell-survival fractions for HSG and V79 cells. Note that Table 1 also shows the parameters for the conventional MK model. For respective cell lines, similar parameter values are determined between the modified SMK model and the MK model, except for $R_n$. Table 1 also shows the mean square deviation given by [Equation 25] per data point for each cell line and model. $\alpha_0$, $\beta_0$, $r_d$, $R_n$, and $z_0$ determined as the modified SMK-model parameters (fixed parameters) are stored in the storage unit 25 as a part of the pencil beam source data 41. Note that $X^{2/n}$ in the table indicates the accuracy of fitting and is not included in the fixed parameters.

[Table 1]

| | Human salivary gland tumor cells | | V79 cells | |
|---|---|---|---|---|
| | Modified-SMK model | MK model | Modified-SMK model | MK model |
| $\alpha_0[Gy^{-1}]$ | 0.174 | 0.150 | 0.103 | 0.136 |
| $\beta_0[Cy^{-2}]$ | 0.0568 | 0.0593 | 0.0210 | 0.0200 |
| $r_d$ [$\mu$m] | 0.28 | 0.29 | 0.23 | 0.24 |
| $R_n$ [$\mu$m] | 8.1 | 3.9 | 8.1 | 3.6 |
| zo [Gy] | 66.0 | 55.0 | 122.0 | 105.8 |
| $x^2/n$ | 0.0229 | 0.0452 | 0.0430 | 0.0575 |

Next, using the fact that $\alpha_0$, $\beta_0$, $z_0$, and $r_d$ are determined for each cell species, how much energy is delivered with respect to the kinetic energy of particles (or particle irradiation speed) is calculated and graphically shown for each of $Z^-_{d,D}$, $Z^{-*}_{d,D}$, and $Z^-_{n,D}$, and this graph is used as table data.

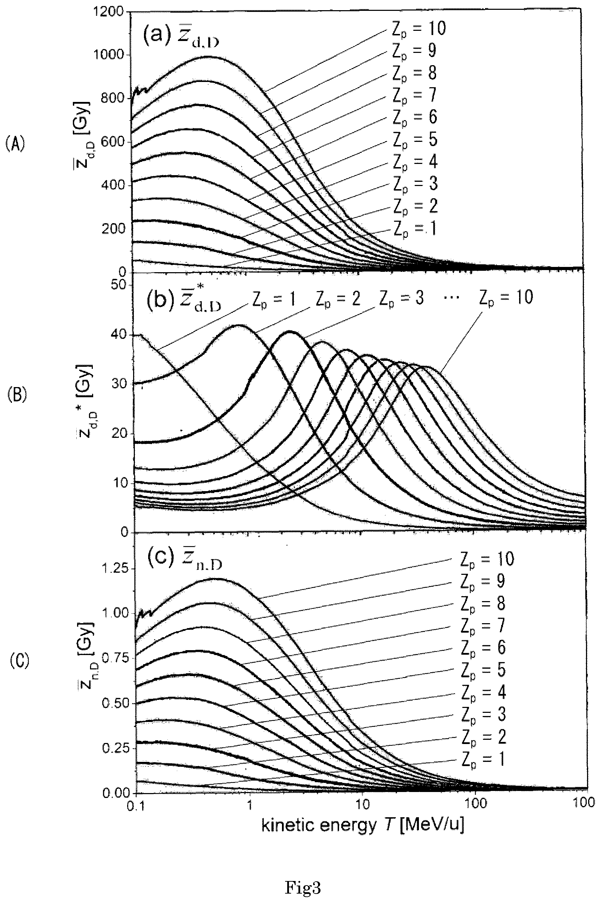

Fig. 3 is an explanatory diagram for describing the graph. Fig. 3(A) shows a graph of $Z^-_{d,D}$ for each nuclide, Fig. 3(B) is a graph of $Z^{-*}_{d,D}$ for each nuclide, and Fig. 3(C) is a graph of $Z^-_{n,D}$ for each nuclide.

As shown in the figure, the relationship between the velocity of the pencil beam and the energy given by the pencil beam is graphically indicated, and using this graph, how much energy is applied when what type of nuclide is radiated at what speed is stored as table data for each of $Z^-_{d,D}$, $Z^{-*}_{d,D}$, and $Z^-_{n,D}$. Note that this table data may be tabular data or an equation that can be calculated each time using a calculation formula.

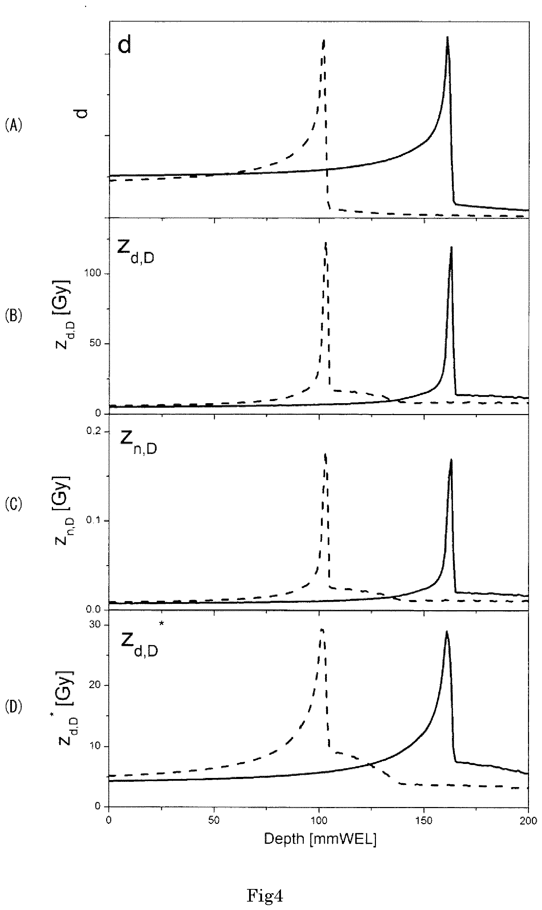

Using each data thus obtained, the integrated dose distribution d (see Fig. 4(A)), $Z^-_{d,D}$ (see Fig. 4(B)), $Z^-_{n,D}$ (see Fig. 4(C)), and $Z^{-*}_{d,D}$ (see Fig. 4(D)) shown in the explanatory diagram of Fig. 4 are obtained. In Figs. 4(A) to 4(D), they are each shown as a function of depth for pencil beams of two types of energies. In Figs. 4(A) to 4(D), the horizontal axis represents depth, the vertical axis represents dose-mean specific energy, the dotted line represents a first beam, and the solid line represents a second beam.

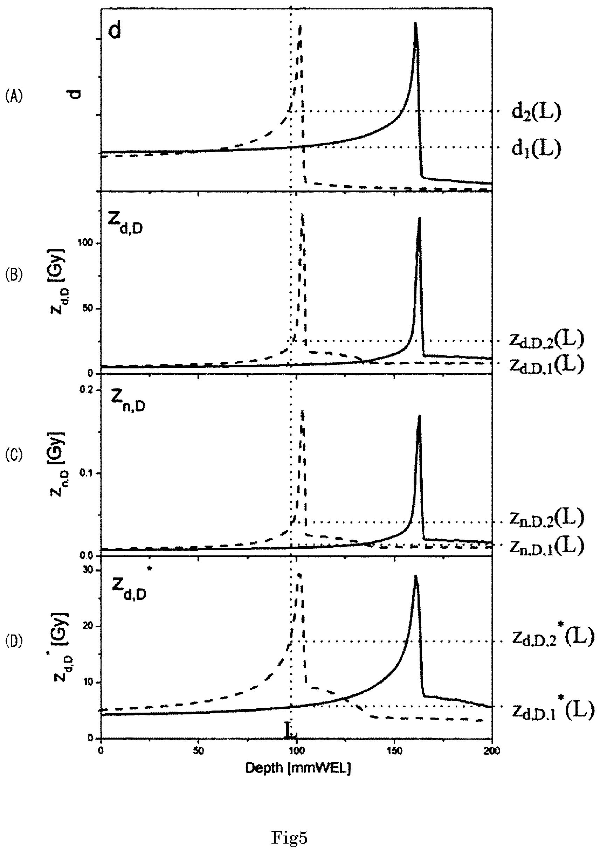

Fig. 5 shows the respective dose-mean specific energies at a certain depth (L) for the abovementioned first beam $d_1$ and second beam $d_2$. That is, Fig. 5 shows the doses $d_1(L)$ and $d_2(L)$ of the respective beams at the depth L in the integrated dose distribution d (see Fig. 5(A)), dose-mean specific energies $Z^-_{d,D1}(L)$ and $Z^-_{d,D2}(L)$ at the depth L for $Z^-_{d,D}$ (see Fig. 5(B)), dose-mean specific energies $Z^-_{n,D1}(L)$ and $Z^-_{n,D2}(L)$ at the depth L for $Z^-_{n,D}$ (see Fig. 5(C)), and dose-mean specific energies $Z^{-*}_{d,D1}(L)$ and $Z^{-*}_{d,D2}(L)$ at the depth L for $Z^{-*}_{d,D}$ (see Fig. 5(D)).

In this way, $Z^-_{d,D}$, $Z^{-*}_{d,D}$, and $Z^-_{n,D}$ of the pencil beams to be superposed in the scanning irradiation method are calculated in advance as a function of depth and stored as pencil beam source data 41.

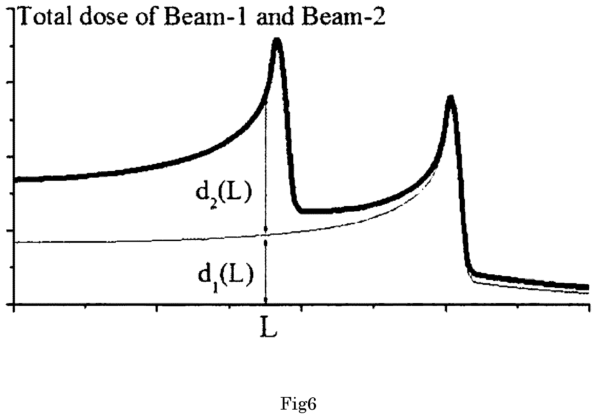

When the first beam and the second beam are superposed, the graph shown in Fig. 6 is obtained.

In Fig. 6, the vertical axis represents dose, and the horizontal axis represents depth. Fig. 6 shows a graph in which the first beam and the second beam are superposed. The following [Equation 43] can be obtained from Fig. 6.

[Equation 43]

$$Z_{d,D}(L)=(d_1(L)z_{d,D,1}(L)+d_2(L)z_{d,D,2}(L))/(d_1(L)+d_2(L))$$

$$Z_{n,D}(L)=(d_1(L)z_{n,D,1}(L)+d_2(L)z_{n,D,2}(L))/(d_1(L)+d_2(L))$$

$$Z_{d,D}*(L)=(d_1(L)z_{d,D,1}^*(L)+d_2(L)z_{d,D,2}^*(L))/(d_1(L)+d_2(L))$$

That is, $Z^-_{d,D}$, $Z^{-*}_{d,D}$, and $Z^-_{n,D}$ of the combined field are calculated by calculating a dose-weighted mean of $Z^-_{d,D}$, $Z^{-*}_{d,D}$, and $Z^-_{n,D}$ applied to the site of interest L by multiple pencil beams. Using $Z^-_{d,D}$, $Z^{-*}_{d,D}$, and $Z^-_{n,D}$ of the combined field, the biological effectiveness (survival fraction) and the relative biological effectiveness (RBE) of the combined field are determined by [Equation 24] described above.

Here, $Z^-_{d,D}$, $Z^{-*}_{d,D}$, and $Z^-_{n,D}$ are measurable physical quantities. Therefore, by measuring $Z^-_{d,D}$, $Z^{-*}_{d,D}$, and $Z^-_{n,D}$ at each position of the combined field, the RBE (relative biological effectiveness) at that position can be predicted from the measured value using the theory of the SMK model.

In this way, the RBE of the combined field can be obtained. $Z^-_{d,D}$ (see Fig. 4(B)), $Z^-_{n,D}$ (see Fig. 4(C)), and $Z^{-*}_{d,D}$ (see Fig. 4(D)) in water determined for each pencil beam using this graph data are registered as the pencil beam source data 41.

Next, the computation by the computation unit 33 will be described in detail.

First, the operator inputs what position and how much survival fraction are intended by beam irradiation. In general, the operator does not directly input the survival fraction, but inputs a clinical dose equivalent to the survival fraction, and the input clinical dose is replaced with or converted to the survival fraction to be used for computation. At this time, the operator also inputs the beam direction and types of nuclides to be used.

Using the abovementioned [Equation 24] and the fixed parameters ($\alpha_0$, $\beta_0$, $z_0$, $r_d$) of the nuclide to be radiated, the computation unit 33 calculates how much survival fraction is obtained when what amount of the nuclide is radiated to the input position.

The computation unit 33 repeats this calculation while changing the beam dose and the nuclide, and calculates which nuclide is radiated to which position at which dose, in order to obtain the best result with respect to the inputted prescription data. Then, the computation unit 33 determines the optimum irradiation parameters by combining a plurality of designated nuclides. An appropriate conventional method is used as the method for determining the optimum irradiation parameter by iterative calculation.

The irradiation planning device 20 transmits the irradiation parameters thus determined to the control device 10, and the control device 10 performs irradiation of the charged particle beam by the particle beam irradiation system 1 using the irradiation parameters.

With the irradiation planning device 20 described above, the RBE of the combined field can be determined from measurable physical quantities, and the biological effectiveness of the combined field can be predicted without conducting a cell irradiation experiment. This makes it possible to accurately predict the RBE of the combined field in a short time and determine the irradiation parameter. In addition, based on the stochastic SMK model, the biological effectiveness and biological doses for high-LET and high-dose heavy-ion therapeutic fields can be calculated, and irradiation plans and treatment plans can be created, in a short time without extending the computational time. In addition, the biological effectiveness of the heavy-ion therapeutic field can be confirmed from the measured values on the basis the theory of the SMK model.

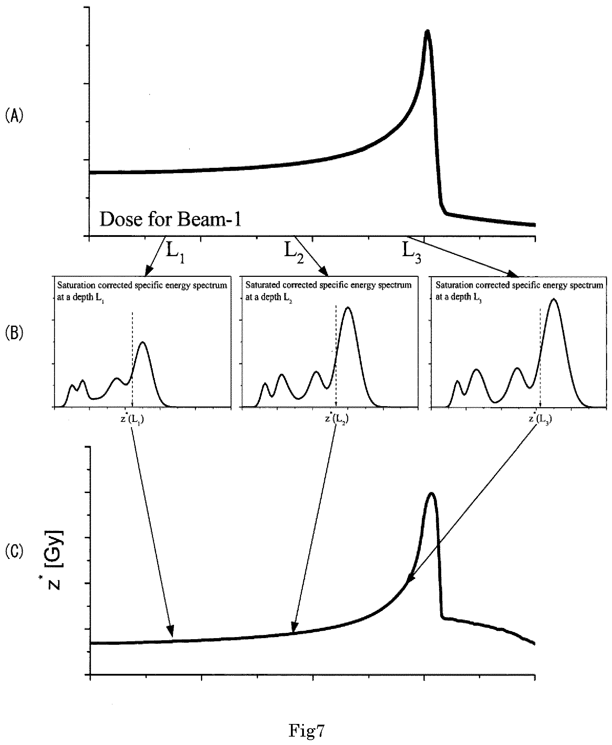

Further, as shown in the explanatory diagram of Fig. 7, with respect to the pencil beam to be superposed in the scanning irradiation shown in Fig. 7(A), a saturation dose-mean specific energy at each depth is obtained from the specific energy spectrum at each depth in which the excessive killing effect is corrected as shown in Fig. 7(B), and the obtained specific energy is registered in the storage unit 25 as the beam axis direction component of the pencil beam source data 41 together with the integrated dose distribution as shown in Fig. 7(C).

In addition, the particle beam irradiation system 1 using the irradiation planning device 20 can accurately predict the biological effectiveness of a high-LET high-dose irradiation field, thereby implementing short-term irradiation with a large dose using high-LET radiation such as oxygen and neon as well as carbon, and being capable of shortening the treatment period.

Further, the particle beam irradiation system 1 can radiate not only a pencil beam of a heavy particle (carbon, oxygen, neon, etc.) but also a pencil beam of a light particle (helium, proton, etc.) in combination. In this case, it is also possible to make a highly accurate prediction in a short time and create an appropriate treatment plan.

Further, since it is not necessary to derive each specific energy spectrum at a site of interest in the iteration calculation of interactive approximation, the computational time and the used area of the memory (used area of RAM of the control device 23) can be significantly reduced. Therefore, it is possible to create the irradiation plan, which is made by the iteration calculation of interactive approximation, within a permissible time in actual medical practice.

Note that the present invention is not limited to the configurations of the abovementioned embodiment, and can be embodied in various modes.

INDUSTRIAL APPLICABILITY

The present invention can be used for a charged particle irradiation system that accelerates a particle beam by an accelerator and radiates the accelerated particle beam, and particularly can be used for a charged particle irradiation system using a scanning irradiation method such as a spot scan method and a raster scan method.

REFERENCE SIGNS LIST

| | |
|---|---|
| 1: | Particle beam irradiation system |
| 20: | Irradiation planning device |
| 25: | Storage device |
| 33: | Computation unit |
| 41: | Pencil beam source data |
| $Z^-_{d,D}$: | Domain dose-mean specific energy |
| $Z^-_{n,D}$: | Cell nucleus dose-mean specific energy |
| $Z^{-*}_{d,D}$: | Domain saturation dose-mean specific energy |

**Claims**

1. An irradiation planning device comprising: a storage means for storing data; and a computation means for performing computation, the device creating an irradiation parameter used when a charged particle is radiated as a pencil beam, wherein the storage means stores
   a domain dose-mean specific energy that is a dose-mean specific energy of domains,
   a cell nucleus dose-mean specific energy that is a dose-mean specific energy of cell nuclei including a large number of domains, and
   a domain saturation dose-mean specific energy that is a saturation dose-mean specific energy of the domains, and
   the computation means predicts a biological effectiveness at a site of interest from the domain dose-mean specific energy, the cell nucleus dose-mean specific energy, and the domain saturation dose-mean specific energy applied to the site of interest from the pencil beam, and determines the irradiation parameter on the basis of the biological effectiveness.

2. The irradiation planning device according to claim 1,
   wherein the storage means stores
   a depth-dose profile as source data of the pencil beam, and
   the domain dose-mean specific energy, the cell nucleus dose-mean specific energy, and the domain saturation dose-mean specific energy at each depth.

3. The irradiation planning device according to claim 1 or 2,
   wherein, regarding the prediction of the biological effectiveness, the computation means obtains a dose-weighted mean of the domain dose-mean specific energy, the cell nucleus dose-mean specific energy, and the domain saturation dose-mean specific energy applied to the site of interest from a plurality of the pencil beams, and predicts the biological effectiveness due to irradiation of the pencil beams on the basis of the obtained dose-weighted mean.

4. The irradiation planning device according to claim 3,
   wherein following [Equation 1] is used for the prediction of the biological effectiveness.

[Equation 1]

$$S(D) = \exp\left(-\alpha_{\text{SMK}} D - \beta_{\text{SMK}} D^2\right)\left[1 + D\left\{-\beta_{\text{SMK}} + \frac{1}{2}\left(\alpha_{\text{SMK}} + 2\beta_{\text{SMK}} D\right)^2\right\}\bar{z}_{\text{n,D}}\right]$$

5. The irradiation planning device according to claim 4,
   wherein, regarding the determination of the irradiation parameter, an RBE is determined by calculating a survival fraction at the site of interest from the dose-weighted mean using the Equation 1.

6. An irradiation planning method for determining an irradiation parameter used by an irradiation planning device that includes a storage means for storing data, and a computation means for performing computation, and that creates the irradiation parameter used when a charged particle is radiated as a pencil beam, the method comprising:

   storing, in the storage means,
   a domain dose-mean specific energy that is a dose-mean specific energy of domains,
   a cell nucleus dose-mean specific energy that is a dose-mean specific energy of cell nuclei including a large number of domains, and
   a domain saturation dose-mean specific energy that is a saturation dose-mean specific energy of the domains; and
   predicting a biological effectiveness at a site of interest from the domain dose-mean specific energy, the cell nucleus dose-mean specific energy, and the domain saturation dose-mean specific energy applied to the site of interest from the pencil beam, and determining the irradiation parameter on the basis of the biological effectiveness, by the computation means.

7. A charged particle irradiation system that accelerates a charged particle generated from an ion source by an accelerator and radiates the accelerated charged particle to a target as a pencil beam,
   the system radiating the pencil beam in accordance with an irradiation parameter determined by the irradiation planning device according to any one of claims 1 to 5.

Fig1

Fig2

Fig3

Fig4

Fig5

Fig6

Fig7

<table>
<tr><td colspan="2" align="center"><b>INTERNATIONAL SEARCH REPORT</b></td><td colspan="2">International application No.<br>PCT/JP2019/012590</td></tr>
</table>

**A. CLASSIFICATION OF SUBJECT MATTER**
Int.Cl. A61N5/10(2006.01)i, G21K5/04(2006.01)i

According to International Patent Classification (IPC) or to both national classification and IPC

**B. FIELDS SEARCHED**

Minimum documentation searched (classification system followed by classification symbols)
Int.Cl. A61N5/10, G21K5/04

Documentation searched other than minimum documentation to the extent that such documents are included in the fields searched

| | |
|---|---|
| Published examined utility model applications of Japan | 1922–1996 |
| Published unexamined utility model applications of Japan | 1971–2019 |
| Registered utility model specifications of Japan | 1996–2019 |
| Published registered utility model applications of Japan | 1994–2019 |

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| A | WO 2012/032609 A1 (NATIONAL INSTITUTE OF RADIOLOGICAL SCIENCES) 15 March 2012, entire text, all drawings (Family: none) | 1–7 |
| A | JP 2009-531138 A (HAMPTON UNIVERSITY) 03 September 2009, entire text, all drawings (Family: none) | 1–7 |
| A | WO 2016/014972 A1 (INDIANA UNIVERSITY RESEARCH AND TECHNOLOGY CORPORATION) 28 January 2016, entire text, all drawings & US 2017/0157426 A1 | 1–7 |
| P, A | JP 2018-108284 A (TOSHIBA CORP.) 12 July 2018, entire text, all drawings (Family: none) | 1–7 |

☐ Further documents are listed in the continuation of Box C.    ☐ See patent family annex.

| | |
|---|---|
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 20 June 2019 (20.06.2019) | 02 July 2019 (02.07.2019) |

| Name and mailing address of the ISA/ | Authorized officer |
|---|---|
| Japan Patent Office<br>3-4-3, Kasumigaseki, Chiyoda-ku,<br>Tokyo 100-8915, Japan | |
| | Telephone No. |

Form PCT/ISA/210 (second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **TATSUHIKO SATO ; YOSHIYA FURUSAWA.** Cell survival fraction estimation based on the probability densities of domain and cell nucleus specific energies using improved microdosimetric kinetic models. *Radiation research,* 2012, vol. 178, 341-356
- **LORENZO MANGANARO ; GERMANO RUSSO ; ROBERTO CIRIO ; FEDERICO DALMASSO ; SIMONA GIORDANENGO ; VINCENZO MONACO ; SILVIA MURARO ; ROBERTO SACCHI ; ANNA VIGNATI ; ANDREA ATTILI.** A Monte Carlo approach to the microdosimetric kinetic model to account for dose rate time structure effects in ion beam therapy with application in treatment planning simulations. *Med. Phys.,* 2017, vol. 44, 1577-1589
- **HADA M ; GEORGAKILAS AG.** 2008 Formation of clustered DNA damage after high-LET irradiation: a review. *J. Radial. Res.,* vol. 49, 203-210
- **SUZUKI M ; KASE Y ; YAMAGUCHI H ; KANAI T ; ANDO K.** Relative biological effectiveness for cell-killing effect on various human cell lines irradiated with heavy-ion medical accelerator in chiba (HIMAC) carbon-ion beams. *Int. J Radiat. Oneal. Biol. Phys.,* 2000, vol. 48, 241-250
- **INANIWA T ; FURUKAWA T ; KASE Y ; MATSUFUJI N ; TOSHITO T ; MATSUMOTO Y ; FURUSAWA Y ; NODA K.** Treatment planning for a scanned carbon ion beam with a modified microdosimetric kinetic model. *Phys. Med. Biol.,* 2010, vol. 55, 6721-37
- **CHATTERJEE A ; SCHAEFER H J.** Microdosimetric structure of heavy ion tracks in tissue. *Radial. Environ. Biophys.,* 1976, vol. 13, 215-227
- **KIEFER J ; STRAATEN H.** A model of ion track structure based on classical collision dynamics. *Phys. Med. Biol.,* 1986, 311201-1209
- **KASE Y ; KANAI T ; MATSUMOTO Y ; FURUSAWA Y ; OKAMOTO H ; ASABA T ; SAKAMA M ; SHINODA H.** Microdosimetric measurements and estimation of human cell survival for heavy-ion beams. *Radial. Res.,* 2006, vol. 166, 629-638
- **FURUSAWA Y ; FUKUTSU K ; AOKI M ; ITSUKAICHI H ; EGUCHI-KASAI K ; OHARA H ; YATAGAI F ; KANAI T ; ANDO K.** Inactivation of aerobic and hypoxic cells from three different cell lines by accelerated He-, C- and Ne-ion beams. *Radial. Res.,* 2000, vol. 154, 485-96
- **ASO T ; KIMURA A ; KAMEOKA S ; MURAKAMI K ; SASAKI T ; YAMASHITA T.** Geant4 based simulation framework for particle therapy system. *Nuclear Science Symposium IEEE, Conference Record,* 2007, vol. 4, 2564-2567
- **AGOSTINELLI S et al.** Geant4 - a simulation toolkit. *Nucl. Jnstrum. Methods. Phys. Res. A,* 2003, vol. 506, 250-303
- **INANIWA T ; KANEMATSU N ; NODA K ; KAMADA T.** Treatment planning of intensity modulated composite particle therapy with dose and linear energy transfer optimization. *Phys. Med. Biol.,* 2017, vol. 62, 5180-97
- **INANIWA T ; KANEMATSU N.** A trichrome beam model for biological dose calculation in scanned carbon-ion radiotherapy treatment planning. *Phys. Med. Biol.,* 2015, vol. 60, 437-451